# FASCICULE DE BREVET EUROPEEN

(11) **EP 2 164 936 B1**
(45) Date de publication et mention de la délivrance du brevet: **10.11.2010**
(21) Numéro de dépôt: 08826122.7
(22) Date de dépôt: 09.06.2008
(51) Int. Cl.: C11C 3/00, C11C 3/10, C07C 67/02

(54) **AMÉLIORATION DE LA SÉPARATION DANS UN PROCÈDE DE PRODUCTION D'ESTERS ACRYLIQUES À PARTIR D'HUILE VÉGÉTALE OU ANIMALE ET D'UN MONOALCOOL ALIPHATIQUE.**
TRENNUNGSVERBESSERUNG IN EINEM VERFAHREN ZUR HERSTELLUNG VON ACRYLESTERN AUS PFLANZEN- ODER TIERÖL UND EIN ALIPHATISCHER MONOALKOHOL
SEPARATION IMPROVEMENT IN A METHOD FOR PRODUCING ACRYLIC ESTERS FROM VEGETABLE OR ANIMAL OIL AND AN ALIPHATHIC MONOALCOHOL

(30) Priorité: 29.06.2007 FR 0704715
(43) Date de publication de la demande: 24.03.2010
(73) Titulaire: IFP Energies nouvelles, 92852 Rueil-Malmaison Cedex (FR)
(72) Inventeur: KOUDIL, Abdelhakim, F-69001 Lyon (FR); ROUSSET, Romain, F-69600 Oullins (FR); BOURNAY, Laurent, F-69440 Chaussan (FR); COUPARD, Vincent, F-69120 Vaulx en Velin (FR)
(86) Numéro de dépôt international: PCT/FR2008/000781
(87) Numéro de publication internationale: WO 2009/007528

(56) Documents cités:
- EP-A- 1 352 893
- FR-A- 2 752 242
- GB-A- 2 174 697

## Description

### DOMAINE DE L'INVENTION

L'invention concerne un procédé de fabrication amélioré d'esters alkyliques à partir d'huiles végétales ou animales et d'un monoalcool aliphatique.

### ART ANTÉRIEUR

En vue de leur utilisation comme biocarburant, les esters alkyliques d'huiles végétales sont produits à partir d'huiles végétales issues par exemple de colza, de tournesol, de soja ou même de palme. Mal adaptées à l'alimentation directe des moteurs diesel modernes des véhicules particuliers, les huiles végétales constituées essentiellement de triglycérides doivent être transformées par une réaction de transestérification avec un alcool, par exemple le méthanol ou l'éthanol, introduit en excès pour produire des esters méthyliques d'huiles végétales (EMHV) et de la glycérine.

On entend par glycérol le corps pur de formule chimique C₃H₈O₃ et par glycérine ou phase glycérine, un mélange contenant majoritairement du glycérol et d'autres impuretés, comme par exemple de l'eau, du méthanol, des mono-, di- et tri-glycérides, les mono et di-glycérides étant des triglycérides partiellement convertis par la réaction de transestérification.

Le procédé Esterfip-H^{™} développé par l'IFP permet d'obtenir un biodiesel et une glycérine de très bonne qualité, avec des rendements élevés. Le schéma de principe de ce procédé consiste en deux réacteurs de transestérification à lit fixe utilisant un catalyseur hétérogène solide, fonctionnant en continu et installés en série ce qui permet de maximiser la conversion. L'effluent issu du premier réacteur est soumis à une évaporation partielle pour éliminer l'excès de méthanol introduit, et ainsi favoriser la séparation de la glycérine formée tout en déplaçant favorablement l'équilibre réactionnel pour maximiser la conversion dans le deuxième réacteur. A l'issue de la deuxième réaction de transestérification, la majeur partie de l'excès de méthanol est éliminée par évaporation (plus de 99%) et recyclée. La glycérine insoluble est éliminée par décantation et une ultime étape de purification des esters méthyliques consiste en l'élimination de la glycérine soluble par passage sur une colonne remplie d'un adsorbant sélectif. La teneur en eau dans le milieu réactionnel est contrôlée pour rester inférieure à une valeur limite donnée comme décrit dans le brevet US-6,878, 837 déposé au nom de la Demanderesse.

La norme européenne actuelle sur les biocarburants EN 14 214 impose des teneurs maximales en méthanol, eau, glycérol libre ainsi qu'en mono-, di- et triglycérides : 0,2% masse pour le méthanol, 500 mg/kg pour l'eau, 0,02 % masse de glycérol libre, 0,8% masse en monoglycérides et 0.2% masse en di- et triglycérides.

Le glycérol libre, par opposition au glycérol lié, est défini comme étant une molécule de glycérol totalement détachée de toute chaîne carbonée et de formule C₃H₈O₃.

On parle de glycérol lié lorsque le groupement fonctionnel du glycérol C₃H₈O₃ se retrouve alkylé à une ou plusieurs chaînes d'acides gras donnant des molécules de monoglycérides, diglycérides ou triglycérides.

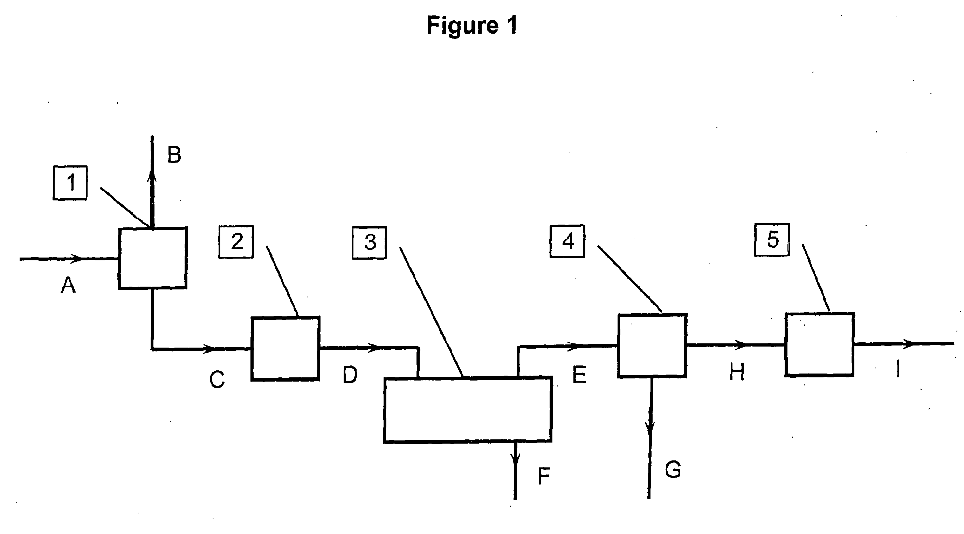

Dans le procédé Esterfip-H™ représenté schématiquement sur la Figure 1 tel que décrit dans l'art antérieur, le flux A, en sortie de section réactionnelle, contient majoritairement des esters méthyliques, du méthanol, du glycérol et des glycérides partiellement convertis (monoglycérides, diglycérides et triglycérides), ainsi que des traces d'eau, impureté présente dans la charge. La conversion atteinte dans cette section réactionnelle (deux étages de réaction avec une étape intermédiaire de séparation de la glycérine coproduite) permet d'obtenir des teneurs en glycérides partiels compatibles avec la norme européenne sur le biodiesel.

Dans le cas particulier du procédé Esterfip-H™, les esters méthyliques et le glycérol sont très peu solubles et le méthanol présent joue le rôle de co-solvant. Donc la teneur en glycérol dans la phase ester est d'autant plus importante que la température est élevée et que la teneur en méthanol est forte.

Par ailleurs, le glycérol pur a une densité proche de 1,2 g.cm⁻³ alors que celle de l'ester se situe autour de 0,9 g.cm⁻³. En présence de peu de méthanol, la phase contenant majoritairement du glycérol est donc plus dense que la phase ester et à ainsi tendance à se placer en dessous de cette dernière sous l'effet de la gravité. La phase ester constitue donc la phase surnageante.

La séparation du méthanol du flux A issu de la section réactionnelle (non représentée sur la figure) se fait par une évaporation en deux étages, le deuxième se faisant sous vide, pour atteindre les teneurs en méthanol et en eau autorisées par la norme (zone (1) sur la Figure 1), le flux B correspondant au méthanol évaporé. Le méthanol agissant comme co-solubilisant des esters méthyliques et du glycérol, cette étape d'évaporation rend insoluble une partie du glycérol présent dans ce flux à une teneur comprise entre 0,1 et 5% masse.

La partie soluble représente à température ambiante 500 à 700 ppm masse, la teneur maximale admissible imposée par la norme européenne étant de 200 ppm masse de glycérol libre. Cela impose donc de séparer à la fois le glycérol insoluble et une partie du glycérol soluble. Cette séparation se fait ensuite en plusieurs étapes.

Un problème important rencontré lors de cette étape d'évaporation de l'excès d'alcool et par conséquent lors de la formation d'un milieu diphasique (phase ester avec des gouttelettes de glycérol dissout et phase glycérine) est la formation de microémulsion.

De façon générale, une microémulsion peut être obtenue par apport d'énergie extérieure, par exemple en appliquant un très fort cisaillement à un mélange de deux liquides, ou en appliquant une onde ultrasonore au mélange.

Une microémulsion peut également apparaître de façon spontanée (P.Brochette, Emulsification "Elaboration et étude des émulsions" Techniques de l'Ingénieur, traité de Génie des procédés, J 2150), sans aucun apport d'énergie extérieure supplémentaire, lorsque certaines conditions de concentration de chacune des phases du mélange sont remplies. On parle ainsi de nucléation spontanée de la phase discontinue initialement dissoute dans la phase continue.

Dans le cas du système ternaire phase ester/glycérol/alcool que l'on a en sortie de section réactionnelle du procédé Esterfip-H™, l'alcool qui est du méthanol joue le rôle de co-solubilisant. La disparition progressive de l'alcool induite par une évaporation contrôlée conduit à une sursaturation locale de la phase ester en glycérol. Le glycérol en excès de la saturation se dépose autour de ce qu'on appelle des noyaux constitués de tensioactifs : ce phénomène constitue la nucléation. Les monoglycérides présents dans l'effluent jouent le rôle de tensioactifs (« Synthesis of Surfactants from Vegetable Oil Feedstocks ». R.A. Holser, chp 10, Industrial Uses of Vegetable Oils, AOCS Press, 2005). La présence de composés tensioactifs, même en très faible quantité (de l'ordre du ppm) et les faibles tensions d'interface conduisent à la formation de très petites gouttelettes micrométriques. Cette création de gouttelettes a lieu sans avoir besoin d'aucune autre énergie que celle qui tend à rattraper un équilibre ternaire rompu du fait de la disparition progressive mais pas nécessairement complète de l'un des trois composés du mélange ternaire, ici le méthanol.

La formation de microémulsions entraîne la présence d'une population importante de microgouttelettes de glycérol dispersé et dissout dans la phase ester.

Dans le procédé Esterfip-H™ tel que décrit dans l'art antérieur, la séparation de la phase glycérine se fait par décantation gravitaire dans un ballon décanteur. Cette étape consiste à envoyer ce flux dans un ballon décanteur 3 dont la fonction est de permettre aux gouttelettes de phase glycérine, plus denses que la phase ester de tomber sous l'action de la gravité.

De façon générale, la taille du ballon décanteur et le temps de séjour de la charge dans cet appareillage définissent le seuil de coupure du décanteur. Le seuil de coupure s'exprime en µm et correspond à la taille minimale des gouttes qu'il est possible de séparer par décantation dans le ballon. En dessous de ce seuil, les gouttelettes ne décantent pas suffisamment rapidement dans le décanteur et sont entraînées avec la phase ester dans les étapes suivantes du procédé. Or des temps de décantation trop longs nécessitent une immobilisation plus importantes des effluents, entraînant donc des sur-stockages coûteux et des pertes au niveau de la rentabilité du procédé.

Si le seuil de coupure se situe autour de 100 µm, les temps de décantation sont rapides et de l'ordre de moins d'une heure (« Extraction liquide-liquide », Description des appareils, J. Leybros, Techniques de l'ingénieur, Traité génie des procédés, J2764). Si le seuil de coupure est inférieur à 10 µm, les temps de décantation deviennent très longs et le coût de l'installation est nettement augmenté.

Le ballon décanteur peut se présenter sous la forme d'une capacité de forme cylindrique dont l'axe de symétrie est placé horizontalement. Le flux D contenant l'ester avec des gouttes de phase glycérine est injecté à une extrémité du ballon. Deux sorties sont placées à l'extrémité du ballon : l'une située sur la génératrice supérieure pour recueillir la phase ester surnageante, l'autre située au fond du ballon décanteur pour recueillir la phase glycérine. Le flux d'ester contenant les gouttelettes de glycérine va donc cheminer dans le ballon décanteur horizontalement depuis l'entrée vers les sorties à une vitesse dépendant de la section donc du diamètre de ce ballon. Pendant ce cheminement horizontal, les gouttes de glycérine, sous l'effet de la gravité ont tendance à tomber vers le fond du ballon décanteur où elles coalescent, c'est à dire qu'elles se regroupent pour former une phase glycérine continue qui peut être soutirée (flux F). La phase ester appauvrie en gouttes de glycérine est soutirée sur le dessus du ballon (flux E).

Lorsque le phénomène de formation de microémulsion qui a lieu lors de l'étape d'évaporation de l'alcool est important, la séparation par décantation gravitaire n'est pas suffisante et les gouttelettes de plus petite taille sont tout de même entraînées dans les étapes suivantes du procédé.

Dans le procédé Esterfip-H™, le flux d'esters E sortant du décanteur est envoyé dans un coalesceur (4). Cet équipement permet aux gouttelettes de glycérine dont la taille n'était pas suffisante pour décanter dans l'étape précédente et par conséquent qui ont été entraînées à la sortie du décanteur de se rencontrer pour en former de plus grosses et ensuite décanter efficacement. Le flux de phase glycérine G est soutiré en point bas du coalesceur. En théorie à la sortie de cet équipement, le flux d'esters H ne contient plus de glycérine insoluble. Toutefois, une teneur trop importante en glycérine entraînée en sortie de décanteur en amont augmente la difficulté de la coalescence et nécessite l'utilisation d'un appareillage de plus grande dimension. Sa réalisation nécessitera donc une quantité d'acier plus importante et des outillages de plus grande dimension. Par conséquent, il sera plus coûteux.

Les coalesceurs sont des systèmes qui permettent d'agrandir la taille des fines gouttelettes en favorisant le phénomène de coalescence c'est à dire la formation de gouttelettes plus grosses (Perry 's Chemical Engineers' Handbook , 7th Edition, Chp 15-17 "Liquid-liquid extraction equipment"). Les gouttelettes une fois devenues plus grosses peuvent plus facilement être séparées par décantation par exemple. Les coalesceurs sont des lits de solide fibreux ou poreux dont les propriétés sont choisies en fonction du système à séparer. Généralement, ce sont des fibres de coton et de verre qui sont utilisées.

Comme tout matériel industriel, le coalesceur ne réalise pas une séparation parfaite ou peut être amené à fonctionner en marche dégradée (débit très important, viellissement, encrassement etc...). Une partie des fines gouttelettes peut passer à travers le milieu coalescent. Cette part sera d'autant plus importante que le nombre de gouttelettes de petites tailles en entrée de l'équipement sera important.

Pour atteindre la teneur admise par la spécification carburant, il est encore nécessaire de séparer la glycérine dissoute dans la phase ester. Cette étape a lieu dans la zone (5) par adsorption sur des solides, par exemple sur des résines échangeuses d'ions. Ces solides fonctionnent en alternant des cycles d'adsorption et de régénération. À l'issue de cette étape, la teneur en glycérine de la phase ester I répond ainsi à la spécification carburant (inférieure à 200 ppm).

La chaîne de traitement de l'ester final comprend donc un décanteur (3) pour séparer la majorité de la glycérine, un coalesceur (4) destiné la glycérine résiduelle insoluble et une zone (5) d'adsorption sur solides pour séparer la glycérine dissoute de la phase ester I. L'étape principale de séparation a lieu dans le décanteur, alors que les étapes ayant lieu dans le coalesceur ou dans la zone d'adsorption sur solides représentent des étapes de finition.

Dans le procédé Esterfip-H™ tel que décrit dans l'art antérieur, les solides situés dans la zone d'adsorption (5), par exemple les résines échangeuses d'ions se trouvent en contact d'une partie de la glycérine insoluble. Or leur utilisation sera d'autant plus optimisée que la part du glycérol insoluble à séparer de la phase ester sera faible. En présence d'une trop grande quantité de glycérol, les solides utilisés dans la zone d'adsorption ont donc tendance à se saturer plus rapidement. La fréquence des cycles adsorption /régénération augmente. La régénération se fait en utilisant un solvant, de préférence du méthanol. Or l'alternance répétée de ces cycles diminue considérablement la durée de vie de ces solides. Pour un fonctionnement optimisé des solides, en sortie du coalesceur, le flux H ne doit contenir que 500 à 700 ppm masse de glycérol soluble.

La présente invention propose donc un schéma de procédé simple et amélioré, qui remédie aux inconvénients précités et dans lequel l'efficacité de la séparation de la glycérine est améliorée en évitant la formation de microémulsions lors de l'étape d'évaporation. Ainsi l'efficacité de l'étape de décantation est améliorée. Or cette étape conditionne précisément le dimensionnement des installations nécessaires pour les étapes suivantes du procédé. Ainsi augmenter l'efficacité du décanteur permet de réduire la taille du coalesceur et d'augmenter son efficacité. La quantité ou la durée de cycle des solides utilisés dans la zone d'adsorption, par exemple les résines échangeuses d'ions s'en trouve ainsi augmentées.

### RÉSUMÉ DE L'INVENTION

La présente invention décrit un procédé de production d'esters alkyliques d'huiles végétales ou animales et de glycérine dans lequel la séparation entre la phase ester et la glycérine est nettement améliorée. La population de gouttelettes de glycérine de petite taille est considérablement réduite grâce à une étape d'addition d'une phase glycérine avant l'étape d'évaporation de l'excès d'alcool. L'addition d'une phase glycérine empêche ainsi l'apparition de microémulsion et par conséquent facilite l'étape de décantation.

L'invention décrit l'installation dans laquelle le procédé de production d'esters alkyliques d'huiles végétales ou animales et de glycérine avec une séparation améliorée entre la phase ester et la glycérine est mis en oeuvre.

### DESCRIPTION DES DESSINS

La Figure 1 donne une représentation schématique d'une partie du procédé Esterfip-H™ tel que décrit dans l'art antérieur.

La Figure 2 donne une représentation schématique d'une partie procédé Esterfip-H™ comprenant l'amélioration proposée selon la présente invention.

### DESCRIPTION DÉTAILLÉE DE L'INVENTION

La présente invention décrit un procédé de production d'esters alkyliques d'acides gras et de glycérine mettant en oeuvre dans une section réactionnelle un ensemble de réactions de transestérification entre une huile végétale ou animale et un monoalcool aliphatique, et utilisant un catalyseur solide hétérogène, comprenant :
a) une étape d'addition d'une phase glycérine de pureté supérieure à 50% masse dans l'effluent issu de la section réactionnelle comprenant des esters alkyliques, du glycérol, des triglycérides partiellement convertis et de l'alcool, puis
b) une étape d'évaporation de l'excès d'alcool,
c) une étape de refroidissement, et
d) une étape de décantation de la phase glycérine et obtention d'une phase ester surnageante.

L'étape d'addition de la phase glycérine est précédée d'une étape de pré-évaporation d'une partie de l'excès d'alcool.

A l'issue de l'étape de décantation, la phase ester obtenue peut être envoyée vers une ou plusieurs étapes de séparation de la glycérine résiduelle. Avantageusement, cette séparation ultérieure a lieu d'abord dans un coalesceur, d'où on extrait une phase glycérine et une phase ester très appauvrie en glycérine insoluble. La phase ester est ensuite envoyée dans au moins une zone d'adsorption sur solides pour séparer la glycérine soluble et ainsi obtenir une phase ester aux spécifications carburant.

Le procédé selon la présente invention permet ainsi d'améliorer notablement la séparation de la glycérine. Ceci est dû au fait que le procédé selon la présente invention permet d'éviter l'apparition de microémulsion lors de la phase d'évaporation de l'excès d'alcool et par conséquent de diminuer considérablement la population de microgouttelettes dont la séparation par décantation gravitaire imposerait des équipements de très grandes dimensions.

Selon le procédé de l'invention, le front de décantation séparant la glycérine de la phase ester avance donc plus vite en présence de glycérine ajoutée. En d'autres termes, l'invention permet d'optimiser l'étape de décantation (taille du décanteur, temps de séjour, production) et également les éventuelles étapes ultérieures de séparation de glycérine résiduelle en fonction des besoins de l'exploitant.

En introduisant une phase glycérine « libre » dans l'effluent issu de la section réactionnelle, on limite les sursaturations locales de la phase ester en glycérol qui sont à l'origine des microémulsions.

Cet apport de phase glycérine peut être réalisé de deux manières :
- soit on injecte, avant l'évaporation de l'alcool, une phase glycérine « libre » de sorte à se situer dans la zone de coexistence de deux phases dans le diagramme ternaire ester/glycérol/alcool. Le glycérol en sursaturation dans la phase ester du fait de la disparition progressive de l'alcool se met nécessairement dans la phase glycérine préexistante, ce qui évite ainsi la formation d'une quelconque microémulsion.
- soit on injecte de la glycérine « libre » au plus près des évaporateurs, de sorte à se situer dans une zone monophasique du diagramme ternaire ester/glycérol/alcool. Une fois dans l'évaporateur, la glycérine libérée du fait de l'évaporation de l'alcool va permettre de se situer dans la zone de coexistence des deux phases, sans former de microémulsions.

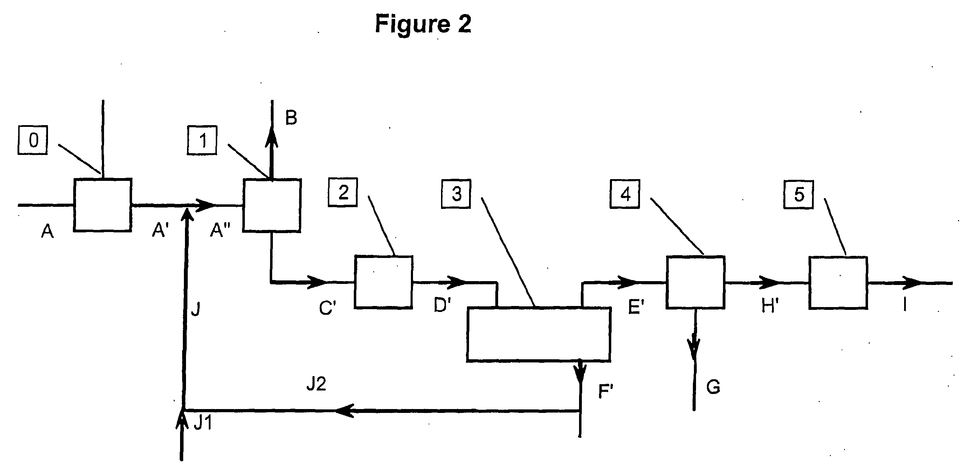

La Figure 2 représente un schéma d'un mode de réalisation particulier du procédé amélioré de la présente invention.

L'installation dans laquelle ce mode de réalisation est mis en oeuvre comprend :
- une section réactionnelle (non représentée sur la figure) en sortie de laquelle on obtient un effluent comprenant des esters alkyliques, du glycérol, des triglycérides partiellement convertis et de l'alcool,
- éventuellement une zone (0) de pré-évaporation d'une partie de l'alcool contenu dans le flux A, en sortie de laquelle on obtient un flux A',
- une conduite par laquelle on injecte une phase glycérine (flux J) de pureté supérieure à 50% masse dans le flux A' appauvri en alcool,
- une zone (1) d'évaporation permettant de séparer l'alcool (flux B) et l'effluent comprenant des esters alkyliques, du glycérol et des triglycérides partiellement convertis (flux C'),
- un échangeur thermique (2) permettant de refroidir le flux C', en sortie duquel on obtient un flux D' ,
- un décanteur (3) permettant de séparer la phase ester surnageante (flux E') et la phase glycérine (flux F'), et
- éventuellement une zone (4,5) de séparation de la glycérine résiduelle.

Avantageusement, cette zone de séparation peut être un coalesceur (4) pour séparer la glycérine insoluble résiduelle et/ou une zone d'adsorption (5) sur solides pour séparer de la phase ester le glycérol soluble.

Le flux A est issu de la section réactionnelle et est majoritairement constitué d'esters alkyliques, d'alcool, de glycérol et de triglycérides partiellement convertis.

L'étape réalisée dans la zone (0) est une étape optionnelle de pré-évaporation d'une partie de l'excès d'alcool. Cette étape peut être réalisée sans générer de phase glycérine insoluble et permet d'obtenir un flux A' comprenant des esters alkyliques, du glycérol, des triglycérides partiellement convertis et une quantité moindre d'alcool.

Un flux J, enrichi en glycérine, est ensuite mélangé au flux A'. Ce mélange constitue le flux A" qui est envoyé dans la zone d'évaporation (1) de l'alcool. En sortie de cette zone, on obtient un flux B correspondant à l'alcool évaporé et un flux C' comprenant des esters alkyliques, du glycérol et des triglycérides partiellement convertis.

Ce flux J représente 0,1 à 100% volumique du débit du flux A issu de la section réactionnelle, ou du flux A' si une étape de pré-évaporation a eu lieu préalablement. Préférentiellement, il représente 1 à 50% volumique du débit du flux A.

Le flux J contient au moins 50% masse de glycérol et préférentiellement 75% masse et de façon encore plus préférée 90% masse.

La glycérine utilisée pour cette étape d'addition est constituée des flux J1 et/ou J2.

Le flux J1 correspond à une phase glycérine provenant d'une source extérieure et ayant une pureté supérieure d'au moins 50% masse, de préférence supérieure à 70% masse, et encore plus préférentiellement supérieure à 90% masse.

Le flux J2 correspond à une partie du flux F' et est constitué de glycérine de pureté d'au moins 50% masse, de préférence supérieure à 75% masse et encore plus préférentiellement supérieure à 90% masse, directement soutirée du décanteur (3)

Très préférentiellement, le flux J provient en totalité du recycle (flux J2) de la glycérine soutirée du décanteur (3) et représente une partie du flux F'.

Le flux C' est ensuite soumis à un refroidissement dans un échangeur thermique (2), dans le but de diminuer la teneur en glycérol dissout dans la phase ester.

Lors de l'étape d'évaporation de l'alcool et de refroidissement du flux comprenant des esters alkyliques, du glycérol et des triglycérides partiellement convertis, la glycérine rendue insoluble va venir grossir l'inventaire de phase glycérine déjà présente correspondant à la quantité injectée par le flux J. Ainsi, le flux D' sortant de l'échangeur thermique (2) contiendra une quantité plus importante de glycérine, sous forme de gouttes plus grosses qui seront ensuite facilement décantées dans l'étape de séparation de la phase ester et de la phase glycérine.

Le flux D' est ensuite envoyé dans le ballon décanteur (3), dans lequel a lieu l'essentiel de la séparation des deux phases (ester et glycérine).

Un avantage de la présente invention est de diminuer la taille du décanteur grâce à l'augmentation de la taille des gouttelettes.

Au fond du décanteur, on extrait un flux F' de glycérine, alors que la phase ester est soutirée sur le dessus du ballon (flux E').

Selon le procédé de la présente invention, le flux E', du fait de la décantation améliorée, contient moins de glycérine entraînée dans la phase ester.

Avantageusement, après l'étape de décantation, le flux E' peut être envoyé vers un coalesceur (4). Compte tenu du fait que la décantation de la glycérine est améliorée, cet équipement sera de taille plus réduite. Un flux G' de glycérine est soutiré en point bas du coalesceur.

Le flux H' d'esters ne contenant pratiquement plus et de préférence plus du tout de glycérine insoluble extrait du coalesceur (4) peut avantageusement être envoyé vers la zone (5) pour une étape d'adsorption sur des solides. Lors de cette étape, l'alternance des cycles adsorption /régénération subis par les solides est ainsi moins fréquente et leur durée de vie est donc augmentée. Les phases de régénération du solide sont mises en oeuvre avec une quantité moins importante de solvant. De cette façon, l'économie de la chaîne de traitement s'en trouve grandement améliorée.

Si la décantation permet de séparer toute la glycérine insoluble, on peut par exemple avantageusement envisager d'envoyer le flux d'ester issu du décanteur (3) directement vers une zone (5) d'adsorption sur solides, sans avoir besoin d'une étape supplémentaire de coalescence.

Pour caractériser la population de glycérine dans la phase ester du point de vue de la taille des gouttes, il est nécessaire de disposer de plusieurs méthodes de mesures et/ou de calculs.

Lorsque la phase ester ne contient que du glycérol dissout, elle est complètement limpide. Lorsqu'elle contient des gouttelettes de glycérine insoluble, elle a tendance à devenir trouble. Il est donc possible d'observer visuellement la limite entre la phase trouble et la phase limpide : cette limite est définie comme étant le front de décantation. Le suivi du front de décantation et notamment la vitesse de déplacement de ce front permettent d'estimer la taille des gouttes présentes dans la phase ester. Pour ce faire, un échantillon d'une émulsion constituée de la phase ester continue contenant des gouttelettes de glycérine est disposé dans une éprouvette graduée. En suivant le temps le déplacement du front de décantation, il est possible de calculer une vitesse de déplacement de ce front. A partir de valeurs étalons corrélant la taille des gouttes et la vitesse de déplacement du front de décantation, il est possible d'en déduire les tailles des gouttelettes parmi les plus petites.

Des temps de décantation de l'ordre de plusieurs heures impliquent des tailles de gouttes très petites de l'ordre de quelques microns.

Des mesures de taille des gouttes sont également confirmées par microscopie optique, en disposant des échantillons de l'émulsion sous un microscope optique à grossissement variable. On mesure ainsi directement les différentes tailles de gouttelettes de glycérine coexistant dans le mélange ester-glycérine. Ces tailles ainsi mesurées permettent également de vérifier les résultats obtenus par le suivi du front de décantation.

Des calculs complémentaires de mécanique des fluides numériques permettent de suivre les trajectoires des gouttelettes dans le ballon décanteur. On peut de ce fait faire des estimations de l'efficacité de séparation du décanteur pour les gouttelettes de glycérine rentrant avec le flux d'esters. La taille des gouttelettes étant par ailleurs un paramètre du calcul, il est possible de la faire varier jusqu'à obtenir une séparation correspondant à celle réellement observée sur le décanteur.

## Revendications

1. Procédé de production d'esters alkyliques d'acide gras et de glycérine mettant en oeuvre dans une section réactionnelle un ensemble de réactions de transestérification entre une huile végétale ou animale et un monoalcool aliphatique et utilisant un catalyseur solide hétérogène comprenant :
a) une étape d'addition d'une phase glycérine du pureté supérieure à 50% masse dans l'effluent issu de la section réactionnelle comprenant des esters alkyliques, du glycérol, des triglycérides partiellement convertis et de l'alcool, puis
b) une étape d'évaporation de l'excès d'alcool dudit effluent, puis
c) une étape de refroidissement, et
d) une étape de décantation de la phase glycérine et obtention d'une phase ester surnageante.

2. Procédé selon la revendication 1 dans lequel l'étape d'addition de la phase glycérine est précédée d'une étape de pré-évaporation d'une partie de l'excès d'alcool.

3. Procédé selon l'une des revendications précédentes dans lequel le débit du flux de la phase glycérine additionnée représente 0,1 à 100% volumique du débit du flux.

4. Procédé selon la revendication 3 dans lequel ledit débit représente 1 à 50% volumique du débit du flux.

5. Procédé selon l'une des revendications précédentes dans lequel la phase glycérine additionnée provient en partie du recycle de la glycérine soutirée du décanteur.

6. Procédé selon l'une des revendications 1 à 5 dans lequel la phase glycérine additionnée provient en totalité du recycle de la glycérine soutirée du décanteur.

7. Procédé selon l'une des revendications précédentes dans lequel la glycérine additionnée a une pureté supérieure à 75% masse et préférentiellement supérieure à 90% masse.

8. Procédé selon l'une des revendications précédentes dans lequel ladite phase ester surnageante (flux E') est envoyée dans un coalesceur (4), dont on soutire une phase glycérine en point bas (flux G), et une phase ester (flux H').

9. Procédé selon l'une des revendications précédentes dans lequel la phase ester (flux H') est envoyée vers une zone (5) pour au moins une étape d'adsorption sur des solides.

10. Installation dans laquelle on met en oeuvre le procédé de production d'esters alkyliques d'acide gras et de glycérine selon l'une des revendications 1 à 9 comprenant :
- une section réactionnelle en sortie de laquelle on obtient un effluent (Flux A) comprenant des esters alkyliques, du glycérol, des triglycérides partiellement convertis et de l'alcool, une zone (0) de pré-évaporation d'une partie de l'alcool contenu dans le flux A, en sortie de laquelle on obtient un flux A',
- une conduite par laquelle on injecte une phase glycérine (flux J) de pureté supérieure à 50% masse dans le flux A' appauvri en alcool,
- une zone (1) d'évaporation permettant de séparer l'alcool (flux B) et l'effluent comprenant des esters alkyliques, du glycérol et des triglycérides partiellement convertis (flux C'),
- un échangeur thermique (2) permettant de refroidir le flux C', en sortie duquel on obtient un flux D',
- un décanteur (3) permettant de séparer la phase ester surnageante (flux E') et la phase glycérine (flux F'), et
- éventuellement une zone (4,5) de séparation de la glycérine résiduelle.

11. Installation selon la revendication 10 dans laquelle la zone de séparation comprend un coalesceur (4) et/ou une zone (5) d'adsorption sur solides.

## Claims

1. A method of producing fatty acid alkyl esters and glycerin using, in a reaction section, a set of transesterification reactions between a vegetable or animal oil and an aliphatic monoalcohol, and using a heterogeneous solid catalyst, comprising:
a) a stage of addition of a glycerin phase of purity above 50 % by mass in the effluent from the reaction section comprising alkyl esters, glycerol, partly converted triglycerides and alcohol,
b) a stage of evaporation of the excess alcohol of said effluent,
c) a cooling stage, and
d) a stage of decantation of the glycerin phase and obtaining a supematent ester phase.

2. A method as claimed in claim 1, wherein the glycerin phase addition stage is preceded by a stage of pre-evaporation of part of the excess alcohol.

3. A method as claimed in any one of the previous claims, wherein the flow rate of the stream of added glycerin phase represents 0.1 to 100 % by volume of the flow rate of the stream.

4. A method as claimed in claim 3, wherein said flow rate represents 1 to 50 % by volume of the flow rate of the stream.

5. A method as claimed in any one of the previous claims, wherein the added glycerin phase partly comes from the recycle of the glycerin discharged from the decanter.

6. A method as clamed in any one of claims 1 to 5, wherein the added glycerin phase entirely comes from the recycle of the glycerin discharged from the decanter.

7. A method as claimed in any one of the previous claims, wherein the added glycerin has a purity above 75 % by mass and preferably above 90 % by mass.

8. A method as claimed in any one of the previous claims, wherein said supematent ester phase (stream E') is sent to a coalescer (4) from which a glycerin phase (stream G) is discharged at the bottom point thereof and an ester phase (stream H') is discharged.

9. A method as claimed in any one of the previous claims, wherein the ester phase (stream H') is sent to a zone (5) for at least one solid adsorption stage.

10. An installation wherein the method of producing fatty acid alkyl esters and glycerin as claimed in any one to claims 1 to 9 is implemented, comprising:
- a reaction section at the outlet of which an effluent (stream A) comprising alkyl esters, glycerol, partly converted triglycerides and alcohol is obtained,
- a zone (0) intended for pre-evaporation of part of the alcohol contained in stream A, at the outlet of which a stream A' is obtained,
- a line through which a glycerin phase (stream J) of purity above 50 % by mass is injected into alcohol-depleted stream A',
- an evaporation zone (1) allowing to separate the alcohol (stream B) and the effluent comprising alkyl esters, glycerol and partly converted triglycerides (stream C'),
- a heat exchanger (2) allowing stream C' to be cooled, at the outlet of which a stream D' is obtained,
- a decanter (3) allowing to separate the supematent ester phase (stream E') and the glycerin phase (stream F'), and
- possibly a residual glycerin separation zone (4, 5).

11. An installation as claimed in claim 10, wherein the separation zone comprises a coalescer (4) and/or a solid adsorption zone (5).

## Patentansprüche

1. Verfahren zur Herstellung von Alkylestern aus Fettsäuren und Glycerin, bei dem in einem Reaktionsabschnitt eine Reaktionsfolge zur Umesterung zwischen einem Pflanzen- oder Tieröl und einem aliphatischen Monoalkohol durchgeführt wird, wobei ein heterogener Feststoffkatalysator verwendet wird, umfassend:
a) einen Schritt zum Zugeben einer Glycerinphase mit einer Reinheit von mehr als 50 Masse-% in den Abfluss aus dem Reaktionsabschnitt, der Alkylester, Glycerol, teilweise umgewandelte Triglyceride und Alkohol umfasst, dann
b) einen Schritt zum Verdampfen des Überschussalkohols des Abflusses, dann
c) einen Schritt zum Abkühlen, und
d) einen Schritt zum Dekantieren der Glycerinphase und zum Erhalten einer überstehenden Esterphase.

2. Verfahren nach Anspruch 1, wobei dem Schritt zum Zugeben der Glycerinphase ein Schritt zum Vorverdampfen eines Teils des Überschussalkohols vorausgeht.

3. Verfahren nach einem der vorhergehenden Ansprüche, wobei der Durchflussstrom der zugegebenen Glycerinphase 0,1 bis 100 Vol.-% des Durchflussstroms darstellt.

4. Verfahren nach Anspruch 3, wobei dieser Durchfluss 1 bis 50 Vol.-% des Durchflussstroms darstellt.

5. Verfahren nach einem der vorhergehenden Ansprüche, wobei die zugegebene Glycerinphase teilweise aus der Rückführung des Glycerins stammt, das vom Dekanter abgezogen wird.

6. Verfahren nach einem der Ansprüche 1 bis 5, wobei die zugegebene Glycerinphase vollständig aus der Rückführung des Glycerins stammt, das vom Dekanter abgezogen wird.

7. Verfahren nach einem der vorhergehenden Ansprüche, wobei das zugegebene Glycerin eine Reinheit von mehr als 75 Masse-% und vorzugsweise mehr als 90 Masse-% hat.

8. Verfahren nach einem der vorhergehenden Ansprüche, wobei die überstehende Esterphase (Strom E') in einen Flüssigkeitsabscheider (4) geschickt wird, aus dem am unteren Punkt eine Glycerinphase (Strom G) und eine Esterphase (Strom H') abgezogen werden.

9. Verfahren nach einem der vorhergehenden Ansprüche, wobei die Esterphase (Strom H') zu einer Zone (5) für mindestens einen Schritt zur Adsorption auf Feststoffen geschickt wird.

10. Anlage, in der das Verfahren zur Herstellung von Alkylestern aus Fettsäuren und Glycerin nach einem der Ansprüche 1 bis 9 durchgeführt wird, umfassend:
- einen Reaktionsabschnitt an dessen Ausgang ein Abfluss (Strom A) erhalten wird, der Alkylester, Glycerol, teilweise umgewandelte Triglyceride und Alkohol umfasst,
- eine Zone (0) zum Vorverdampfen eines Teils des Alkohols, der in Strom A enthalten ist, an deren Ausgang ein Strom A' erhalten wird,
- eine Leitung, durch die eine Glycerinphase (Strom J) mit einer Reinheit von mehr als 50 Masse-% in den Strom A', der an Alkohol verarmt ist, injiziert wird,
- eine Zone (1) zum Verdampfen, die es ermöglicht, den Alkohol (Strom B) und den Abfluss, der Alkylester, Glycerol und teilweise umgewandelte Triglyceride umfasst (Strom C'), abzutrennen,
- einen Wärmetauscher (2), der es ermöglicht, den Strom C' abzukühlen, an dessen Ausgang ein Strom D' erhalten wird,
- einen Dekanter (3), der es ermöglicht, die überstehende Esterphase (Strom E') und die Glycerinphase (Strom F') abzutrennen, und
- gegebenenfalls eine Zone (4, 5) zum Abtrennen des restlichen Glycerins.

11. Anlage nach Anspruch 10, wobei die Zone zum Abtrennen einen Flüssigkeitsabscheider (4) und/oder eine Zone (5) zur Adsorption auf Feststoffen umfasst.
